# EUROPÄISCHE PATENTSCHRIFT

(11) **EP 1 850 134 B1**
(45) Veröffentlichungstag und Bekanntmachung des Hinweises auf die Patenterteilung: **30.01.2013**
(21) Anmeldenummer: 07007503.1
(22) Anmeldetag: 12.04.2007
(51) Int. Cl.: G01N 33/86

(54) **Verfahren und Vorrichtung zur Bestimmung der Thrombozytenfunktion und der Flussbedingungen**
Method and Apparatus for the determination of the Thromboyte function and flow conditions
Procédé et dispositif destinés à la détermination de la fonction des thrombocytes et des conditions de flux

(30) Priorität: 28.04.2006 DE 102006020385
(43) Veröffentlichungstag der Anmeldung: 31.10.2007
(73) Patentinhaber: Siemens Healthcare Diagnostics Products GmbH, 35041 Marburg (DE)
(72) Erfinder: Rechner, Andreas, Dr., 35043 Marburg (DE)

(56) Entgegenhaltungen:
- EP-A1- 0 716 744
- WO-A-02/074322
- WO-A-2005/007868
- MISCHKE R ET AL: "Influence of platelet count, acetylsalicylic acid, von Willebrand's Disease, coagulopathies, and haematocrit on results obtained using a platelet function analyser in dogs." VETERINARY JOURNAL, Bd. 165, Nr. 1, Januar 2003 (2003-01), Seiten 43-52, XP002437870 ISSN: 1090-0233
- BIN XU ET AL: "Acyclic Analogues of Adenosine Biphosphates as P2Y Receptor Antagonists: Phosphate Substitution Leads to Multiple Pathways of Inhibition of Platelet Aggregation" JOURNAL OF MEDICINAL CHEMISTRY, AMERICAN CHEMICAL SOCIETY. WASHINGTON, US, Bd. 45, 2002, Seiten 5694-5709, XP002285317 ISSN: 0022-2623
- Sigma-Aldrich Prod. No. M5942 MRS2395 Cell Signalling and Neuroscience First P2Y12 Receptor Antagonist available from Sigma-RBI XP002437879
- JUDGE HM, BUCKLAND RJ, STOREY RF: "Incomplete P2Y12 receptor blockade by Clopidogrel is not due to the presence of an internal pool of P2Y12 Receptors" JOURNAL OF THROMBOSIS AND HAEMOSTASIS, Bd. 3, Nr. Supplement1, August 2005 (2005-08), Seite P2148, XP002437871
- WIVIOTT STEPHEN D ET AL: "Randomized comparison of prasugrel (CS-747, LY640315), a novel thienopyridine P2Y12 antagonist, with clopidogrel in percutaneous coronary intervention: results of the Joint Utilization of Medications to Block Platelets Optimally (JUMBO)-TIMI 26 trial." CIRCULATION 28 JUN 2005, Bd. 111, Nr. 25, 28. Juni 2005 (2005-06-28), Seiten 3366-3373, XP002437872 ISSN: 1524-4539
- KAM P C A ET AL: "The thienopyridine derivatives (platelet adenosine diphosphate receptor antagonists), pharmacology and clinical developments." ANAESTHESIA JAN 2003, Bd. 58, Nr. 1, Januar 2003 (2003-01), Seiten 28-35, XP002437873 ISSN: 0003-2409
- KOZEK-LANGENECKER S A ET AL: "[Locoregional anesthesia and coagulation inhibitors. Recommendations of the Task Force on Perioperative Coagulation of the Austrian Society for Anesthesiology and Intensive Care Medicine]" DER ANAESTHESIST MAY 2005, Bd. 54, Nr. 5, Mai 2005 (2005-05), Seiten 476-484, XP002437874 ISSN: 0003-2417
- ANDRÉ PATRICK ET AL: "Anticoagulants (thrombin inhibitors) and aspirin synergize with P2Y12 receptor antagonism in thrombosis." CIRCULATION 25 NOV 2003, Bd. 108, Nr. 21, 25. November 2003 (2003-11-25), Seiten 2697-2703, XP002437875 ISSN: 1524-4539
- HOUSTON DAYLE ET AL: "[P-32]2-iodo-N-6-methyl-(N)-methanocarba- 2 '-deoxyadenosine-3 ',5 '-bisphosphate ([P-32]MRS2500), a novel radioligand for quantification of native P2Y(1) receptors" BRITISH JOURNAL OF PHARMACOLOGY, Bd. 147, Nr. 5, März 2006 (2006-03), Seiten 459-467, XP002437876 ISSN: 0007-1188
- JIN JIANGUO ET AL: "Adenosine diphosphate (ADP)-induced thromboxane A2 generation in human platelets requires coordinated signaling through integrin alphaIIbbeta3 and ADP receptors" BLOOD, Bd. 99, Nr. 1, 1. Januar 2002 (2002-01-01), Seiten 193-198, XP002437877 ISSN: 0006-4971

## Beschreibung

Die Erfindung liegt auf dem Gebiet der Thrombyzytenfunktionsdiagnostik und betrifft ein in vitro-Verfahren zur Bestimmung der Thrombozytenfunktion unter Flussbedingungen sowie eine Vorrichtung für die Durchführung dieses Verfahrens. Das Verfahren eignet sich insbesondere zur Bestimmung der Wirkung von Clopidogrel nach oraler Einnahme und anderer antithrombotisch wirksamer P2Y(12)-Antagonisten. Weiterhin kann mit dem Verfahren auch die Blockierung des zweiten ADP-Rezeptors der Thrombozyten (P2Y(1)-Rezeptor) durch spezifische Antagonisten nachgewiesen werden.

Physiologische Vorgänge, die einerseits die Fluidität des Blutes im Gefäßsystem gewährleisten und andererseits durch die Bildung von Blutgerinnseln die Vermeidung von extravasalen Blutverlusten sicherstellen, werden unter dem Begriff Hämostase zusammengefasst. An der Regulation der Hämostase sind eine Vielzahl von Proteinfaktoren beteiligt sowie auch zelluläre Komponenten, wie z. B. die Thrombozyten (Plättchen). Im Falle einer Gefäßverletzung kommt es zuerst zur Anlagerung von Thrombozyten an das subendotheliale Kollagen. Diese Adhäsion wird durch Adhäsivproteine, wie den von-Willebrand-Faktor (VWF) vermittelt. Während des Adhäsionsvorgangs werden die Thrombozyten aktiviert und schütten Mediatoren aus ihren Granulae aus, wodurch die Aggregation weiterer Thrombozyten sowie eine Verstärkung der Aktivierung induziert wird. Auf diese Weise erfolgt ein primärer Gefäßwandverschluss (primäre Hämostase), der erst durch weitere Reaktionen des plasmatischen Gerinnungssystems (sekundäre Hämostase) stabilisiert wird. Fehlregulationen dieser Prozesse können zu einer Thromboseneigung oder einer Blutungsneigung führen und je nach Schweregrad lebensbedrohliche Folgen haben.

In der Gerinnungsdiagnostik wurden verschiedene in vitro Testverfahren entwickelt, mit deren Hilfe bestimmt werden kann, ob das Blut eines Patienten einwandfrei gerinnen kann oder ob eine Gerinnungsstörung vorliegt. Im Fall einer Gerinnungsstörung ist es häufig erforderlich, präzisere Kenntnisse über die Ursache der vorliegenden Störung zu erlangen, um die optimalen therapeutischen Maßnahmen auswählen zu können. Eine wichtige Teilfunktion des Gerinnungssystems, die gezielt untersucht werden kann, ist die primäre Hämostase, die wesentlich von der Funktionsfähigkeit der Thrombozyten abhängt.

Verfahren zur Bestimmung der Thrombozytenfunktion werden nicht nur zur Diagnose von erworbenen oder angeborenen Thrombozytenfunktionsstörungen verwendet, sondern auch zur Überwachung antithrombotischer Therapien. Medikamente, die die Aggregation von Thrombozyten hemmen, werden vorwiegend zur Prophylaxe und Therapie arterieller thromboembolischer Ereignisse, wie Myokardinfarkt oder Schlaganfall eingesetzt. Die verbreitetsten Wirkstoffe mit thrombozytenaggregationshemmender Wirkung sind Acetylsalicylsäure (ASS) und die Thienopyridine Clopidogrel und Ticlopidin. ASS hemmt irreversibel Cyclooxygenase-1 (COX-1), ein intrazelluläres Enzym, das an der Synthese von dem thrombozytenaggregationsfördernden Thromboxan A2 beteiligt ist. Clopidogrel und Ticlopidin gehören aufgrund ihres Wirkungsmechanismus zur Klasse der P2Y(12)-Antagonisten. Nach oraler Einnahme von Clopidogrel oder Ticlopidin entstehen in der Leber Metabolite, die selektiv den P2Y(12)-Rezeptor blockieren. Der purinerge P2Y(12)-Rezeptor wird auf der Thrombozytenoberfläche exprimiert und ist durch extrazelluläres Adenosin-5'-diphosphat (ADP) aktivierbar. Infolge der Aktivierung des P2Y(12)-Rezeptors werden in den Thrombozyten intrazelluläre Prozesse, wie zum Beispiel die Inhibierung der Bildung von cAMP, in Gang gesetzt, die eine Thrombozytenaggregationsreaktion hervorrufen. P2Y(12)-Antagonisten blockieren die P2Y(12)-Rezeptoren auf der Thrombozytenoberfläche und wirken daher antithrombotisch.

Der zweite purinerge ADP-Rezeptor P2Y(1) wird ebenfalls auf der Thrombozytenoberfläche exprimiert und durch extrazelluläres Adenosin-5'-diphosphat aktiviert. Infolge der Aktivierung des P2Y(1)-Rezeptors werden in den Thrombozyten intrazelluläre Prozesse, wie zum Beispiel der Anstieg des intrazellulären Calcium, in Gang gesetzt, die eine Thrombozytenaggregationsreaktion hervorrufen. Antagonisten des P2Y(1)-Rezeptors wirken daher diesem Prozess entgegen und wirken antithrombotisch.

Ein genaues Wissen um den Status der Thrombozytenfunktion von Patienten, die eine antithrombotische Therapie erhalten, wird als immer wichtiger erachtet, da z. B. das Auftreten einer sogenannten Clopidogrelresistenz als ernst zu nehmender Risikofaktor betrachtet wird. Eine Clopidogrelresistenz liegt vor, wenn die Thrombozytenfunktion eines Patienten durch die Gabe der Standarddosis Clopidogrel nicht oder kaum beeinflusst wird. Durch die Bestimmung der Thrombozytenfunktion kann einerseits überprüft werden, ob durch die gewählte Dosierung tatsächlich eine ausreichende antithrombotische Wirkung erzielt wird. Andererseits können zu hohe Dosierungen oder Wirkungen eines antithrombotischen Medikaments festgestellt und behandelt werden, was z. B. vor operativen Eingriffen zum Ausschluss von Blutungskomplikationen notwendig ist.

Im Stand der Technik sind verschiedene Verfahren zur Untersuchung der Thrombozytenfunktion bekannt. Bei der Blutungszeitbestimmung handelt es sich um einen in vivo-Globaltest, der die primäre Hämostase erfasst. Die Blutungszeit wird bestimmt, indem dem Patienten eine kleine Schnitt- oder Stichverletzung zugefügt wird und die Zeit bis zum Blutungsstillstand gemessen wird. Es handelt sich um einen schwer standardisierbaren, grob informativen Test, der vor allem in Notfallsituationen angewendet wird, um einen Überblick über die primäre Hämostase zu erhalten. Die Einnahme von Thrombozytenaggregationshemmern führt zu einer Verlängerung der Blutungszeit. Nachteilig an der Blutungszeitbestimmung ist, dass bei einer normalen Blutungszeit eine Thrombozytenfunktionsstörung nicht ausgeschlossen werden kann.

Verschiedene in vitro-Verfahren ermöglichen einen wesentlich empfindlicheren Nachweis von Thrombozytenfunktionsstörungen. Bei diesen Verfahren wird üblicherweise in einer Vollblutprobe oder in einer Probe von plättchenreichem Plasma (PRP) die Aggregation der Thrombozyten durch Zugabe eines Aktivators induziert und die Aggregationsreaktion gemessen. Die meistverwendeten Aktivatoren, die zur Induktion der Thrombozytenaggregation verwendet werden, sind: ADP (Adenosin-5'-diphosphat), Kollagen, Epinephrin (Adrenalin), Ristocetin und verschiedene Kombinationen davon sowie Thrombin, TRAP (Thrombin-Receptor-Activating-Protein) oder Serotonin.

Bei der Lichttransmissionsaggregometrie, die auch als Plättchenaggregation nach Born bezeichnet wird, wird die Aggregationsfähigkeit der Thrombozyten im plättchenreichen Plasma in Gegenwart von aggregationsinduzierenden Substanzen in einem Aggregometer photometrisch gemessen. Durch die Aggregatbildung wird die Lichtdurchlässigkeit der PRP-Probe erhöht, so dass durch die Messung der Lichtdurchlässigkeit z. B. die Geschwindigkeit der Aggregatbildung bestimmt werden kann. Mit Hilfe der Lichttransmissionsaggregometrie können auch therapeutische Effekte von medikamentös eingesetzten Thrombozytenaggregationshemmern erfasst werden. Ein Nachteil der Lichttransmissionsaggregometrie ist, dass ausschließlich plättchenreiches Plasma als Probenmaterial verwendet werden kann. Plättchenreichem Plasma fehlen nicht nur wichtige Bestandteile des Blutes, wie z. B. die roten und weißen Blutkörperchen, sondern es erfordert außerdem eine zeitaufwändige und fehleranfällige Probenvorbereitung.

BIN XU ET AL ("Acyclic Analogues of Adenosine Biphosphates as P2Y Receptor Antagonists: Phosphate Substitution Leads to Multiple Pathways of Inhibition of Platelet Aggregation" JOURNAL OF MEDICINAL CHEMISTRY, AMERICAN CHEMICAL SOCIETY. WASHINGTON, US, Bd. 45, 2002, Seiten 5694-5709) offenbaren ebenfalls eine Methode zur Detektion der antithrombotischen Wirkung eines P2Y(1)-Antagonisten wie z.B. MRS2395 (2,2-Dimethyl-propionic acid 3-(2-chloro-6-methylaminopurin-9-yl)-2-(2,2-dimethyl-propionyloxymethyl)-propyl ester) mit Hilfe der Messung der Thrombozytenfunktion, wobei die Thrombozytenfunktion mit einem Aggregometer bestimmt wird.

Ein anderes Testprinzip zur Bestimmung der Thrombozytenfunktion ist im Platelet Function Analyzer (PFA-100^{®}, Dade Behring Marburg GmbH, Marburg, Deutschland) verwirklicht. Der PFA-100^{®} ist ein globaler, automatisierter und standardisierter in vitro-Vollbluttest, bei dem die primäre Hämostase unter Flussbedingungen und damit in Gegenwart von hohen Scherkräften gemessen wird. Zur Simulation der Flussbedingungen und der Scherkräfte, wie sie in kleineren arteriellen Blutgefäßen herrschen, wird in einer speziellen Messzelle ein Unterdruck von etwa -40 mbar erzeugt und das Citratvollblut, dass sich in einem Probenreservoir befindet, strömt durch eine Kapillare, die einen Durchmesser von etwa 200 µm hat. Die Kapillare mündet in eine Messkammer die mit einem Trennelement, z. B. einer Membran abgeschlossen ist, welche eine kapillare zentrale Öffnung (Apertur) enthält, durch die das Blut aufgrund des Unterdrucks durchtritt. In den meisten Fällen ist die Membran, zumindest in dem Bereich um die Apertur mit einem oder mehreren Aktivatoren, die die Thrombozytenaggregation induzieren, versetzt, so dass das vorbeiströmende

Blut mit den aggregationsinduzierenden Substanzen im Bereich der Apertur in Kontakt kommt. Infolge der induzierten Adhäsion und Aggregation der Thrombozyten bildet sich im Bereich der Apertur ein Thrombozytenpfropf (Thrombus), der die Membranöffnung verschließt und den Blutfluss stoppt. In diesem System wird die Zeit gemessen, die bis zum Verschluss der Membranöffnung benötigt wird. Diese sogenannte Verschlusszeit korreliert mit der Funktionsfähigkeit der Thrombozyten. Eine Messzelle zur Verwendung in einem Verfahren zu Bestimmung der Thrombozytenfunktion anhand der Verschlusszeit ist z. B. in dem Patentdokument WO 97/34698 beschrieben. Bislang werden in dem Verfahren zur Bestimmung der Verschlusszeit Messzellen verwendet, die mit einer Membran ausgestattet sind, die mit Kollagen (Kol) und zusätzlich entweder mit ADP oder mit Epinephrin (Epi) beschichtet ist. Verschiedene Trennelemente sowie deren Herstellung und Verwendung sind z. B. in dem Patentdokument EP 716 744 B1 beschrieben.

MISCHKE R ET AL ("Influence of platelet count, acetylsalicylic acid, von Willebrand's Disease, coagulopathies, and haematocrit on results obtained using a platelet function analyser in dogs." VETERINARY JOURNAL, Bd. 165, Nr. 1, Januar 2003 (2003-01), Seiten 43-52) beschreiben das PFA-100-System, welches eine biologisch aktive Membran mit einer mikroskopisch grossen Öffnung am Ende einer Kapillare beinhaltet, wobei diese Membran mit Kollagen und ADP beschichtet ist. Beim Hindurchleiten von Blut wird ein Thrombozytenpfropf und damit das Schließen dieser Öffnung induziert. Die Zeit, die für die Bildung des Pfropfs benötigt wird, welcher zur Schließung der Öffnung führt, wird gemessen.

Je nach Ausstattung werden also Kol/ADP-Messzellen und Kol/Epi-Messzellen unterschieden. Üblicherweise wird eine Patientenprobe zunächst mit Hilfe einer Kol/Epi-Messzelle analysiert. Im Falle einer abnormal verlängerten Kol/Epi-Verschlusszeit, die auf eine Störung der Thrombozytenaggregation hinweist, wird im Anschluss eine Kol/ADP-Messung durchgeführt. Ist die Kol/ADP-Verschlusszeit ebenfalls abnormal verlängert, ist dies ein Indikator für eine gestörte Thrombozytenfunktion oder für eine Störung des von-Willebrand-Faktors. Ist die Kol/ADP-Verschlusszeit hingegen normal, so kann dies auf die Gegenwart von Acetylsalicylsäure hinweisen oder auf das Vorliegen einer erworbenen oder angeborenen Thrombozytopathie, wie z. B. der Storage Pool Disease. Ein Nachteil des PFA-100^{®} Systems ist, dass die zur Verfügung stehenden Kol/ADP- und Kol/Epi-Messzellen nur eine begrenzte Empfindlichkeit für den aggregationshemmenden Effekt von Thrombozytenaggregationshemmern aus der Gruppe der Thienopyridine (z. B. Clopidogrel, Ticlopidin) aufweisen. Eine zuverlässige Bestimmung des therapeutischen Effekts von medikamentös eingesetztem Clopidogrel oder Ticlopidin, insbesondere wenn der Patient zusätzlich ASS (z. B. Aspirin^{®}) eingenommen hat, ist mit Hilfe der bekannten Kol/ADP- und Kol/Epi-Messzellen im PFA-100^{®}-System bislang nicht möglich.

Das Patentdokument WO 2005/007868 A2 beschreibt ein anderes Verfahren zur Bestimmung der Thrombozytenfunktion, welches den Nachweis der therapeutischen Wirkung von Clopigogrel und anderen P2Y(12)-Antagonisten ermöglicht. Bei diesem Verfahren wird eine Vollblutprobe eines Patienten mit einem Antikoagulanz gemischt und mit ADP zur Induktion der Thrombozytenaggregation versetzt. Außerdem wird der Probe Prostaglandin E1 (PGE 1) zugegeben. Prostaglandin E1, ein Produkt des menschlichen Arachidonsäuremetabolismus, vermag bereits in geringen Dosen die Reaktivität von Thrombozyten deutlich zu reduzieren und wird daher auch zur Hemmung der Thrombozytenaktivierung eingesetzt. In dem in WO 2005/007868 A2 beschriebenen Testverfahren wird PGE 1 eingesetzt, um die unerwünschte Aktivierung des ADP-Rezeptors P2Y(1) zu verhindern und damit die Spezifität des Testverfahrens für den P2Y(12)-Rezeptor bzw. für P2Y(12)-Antagonisten wie Clopidogrel zu erhöhen. Zusätzlich werden Mikropartikel zugegeben, an die ein Ligand des GPIIb/IIIa-Rezeptors, wie z. B. Fibrinogen gekoppelt ist, und die Aggregationsreaktion wird anhand der zunehmenden Lichtdurchlässigkeit aggregometrisch gemessen. Nachteilig an dem vorbeschriebenen Verfahren ist, dass die Thrombozytenfunktion wie bei der Lichttransmissionsaggregometrie nicht unter dem Einfluss von Flussbedingungen und Scherkräften bestimmt wird.

Der vorliegenden Erfindung lag die Aufgabe zugrunde ein Verfahren zur Bestimmung der Thrombozytenfunktion unter Flussbedingungen bereitzustellen, das es auch ermöglicht, die thrombozytenaggregationshemmende Wirkung von P2Y(12)-Antagonisten zu bestimmen. Die Lösung der Aufgabe besteht in der Bereitstellung der in den Ansprüchen beschriebenen erfindungsgemäßen Verfahren und Gegenstände.

Gegenstand der vorliegenden Erfindung ist ein in vitro-Verfahren zur Bestimmung der Thrombozytenfunktion in einer Vollblutprobe. Bevorzugterweise handelt es sich bei der Vollblutprobe um frisch abgenommenes, venöses, antikoaguliertes menschliches oder tierisches Blut, das innerhalb von vier Stunden nach der Blutentnahme mit Hilfe des erfindungsgemäßen Verfahrens untersucht werden sollte. Bevorzugterweise wird das Vollblut durch Zugabe eines Antikoagulanzes antikoaguliert. Zur Verwendung als Antikoagulanz geeignet sind gepufferte, Calcium-bindende Citratlösungen, wie z. B. 3,2 oder 3,8 %ige gepufferte Natrimcitratlösungen, sowie natürliche oder synthetische direkte Thrombininhibitoren, wie z. B. Hirudin, PPACK (D-Phe-Pro-Arg-Chloromethylketon, HCl), Argatroban und Melagatran, oder natürliche oder synthetische direkte Faktor Xa-Inhibitoren, wie z. B. Antistasin, Tick Anticoagulant Peptide, Yagin, Draculin, GGACK (H-Glu-Glu-Arg-Chloromethylketon), Diamidino-Faktor Xa-Inhibitoren und Mono-Benzamidin Faktor Xa-Inhibitoren.

Das erfindungsgemäße Verfahren zur Bestimmung der Thrombozytenfunktion umfasst mehrere Verfahrensschritte. Zur Simulation der physiologischen Flussbedingungen, wie sie in kleinen Arterien herrschen, wird das Blut, das sich zunächst in einer Haltekammer befindet, durch eine Kapillare geleitet, die bevorzugterweise einen Durchmesser von etwa 200 µm hat. Die Kapillare mündet in eine Messkammer, die von einem Trennelement in zwei Kompartimente unterteilt ist. Das Trennelement weist eine Öffnung auf, durch die das Blut vom ersten in das zweite Kompartiment durchgeleitet wird. Das erfindungsgemäße Verfahren zeichnet sich dadurch aus, dass das verwendete Trennelement einen Aktivator von Adenosin-Rezeptoren, einen Aktivator von intrazellulären Adenylatzyklasen, sowie Calciumionen enthält, wodurch das durch die Öffnung des Trennelements fließende Blut mit diesen im oder auf dem Trennelement enthaltenen Substanzen in Kontakt gebracht wird. Infolge der Thrombozytenaggregation, die durch den Kontakt mit den Substanzen induziert wird, bildet sich ein Thrombozytenpfropf an der Öffnung des Trennelements. Es wird die Zeit gemessen, die für die Bildung des Thrombozytenpfropfs an der Öffnung des Trennelements bis zum Verschluss der Öffnung benötigt wird. Bevorzugterweise wird die Verschlusszeit gemessen, indem ein Gerät verwendet wird, das einen Drucksensor enthält, welcher den Blutfluss durch die Apertur während des Tests bestimmt. Dabei wird nach anfänglicher, schneller Aspiration des Totvolumens der Messzelle zuerst die Initialflussrate ermittelt. Unterschreitet die Flussrate für mehr als 3 Sekunden den 10 % Wert dieser Initialflussrate, wird die Messung beendet und die bis dahin verstrichene Zeit als sogenannte Verschlusszeit angegeben. Diese sogenannte Verschlusszeit, die u. a. abhängig ist von der Aggregationsreaktion der stimulierten Thrombozyten, ist ein Maß für die Thrombozytenfunktion. Bevorzugterweise wird die Verschlusszeit, die für eine Vollblutprobe eines Patienten gemessen wurde, mit einem Verschlusszeitreferenzbereich für Vollblutproben gesunder Probanden verglichen.

Bevorzugterweise wird der Blutfluss durch die Kapillare und durch die Öffnung des Trennelements durch die Schaffung eines Unterdrucks in der Messkammer, also durch Ansaugen erzeugt. In einer besonders bevorzugten Auführungsform wird der Unterdruck durch das Zusammenwirken einer geeigneten Testkartusche und einem Instrument erzeugt. Ein Beispiel für ein derartiges System ist z. B. in dem Patentdokument WO 97/034698 beschrieben.

Das in dem erfindungsgemäßen Verfahren verwendete Trennelement enthält einen Aktivator von Adenosin-Rezeptoren, bevorzugterweise aus der Gruppe Adenosin-5'-diphosphat (ADP), 2-Methylthioadenosin-5'-diphosphat (2-MeSADP) und deren Derivate. In einer bevorzugten Ausführungsform wird ein Trennelement verwendet, das ein ADP-Salz oder ein 2-MeSADP-Salz enthält. In einer bevorzugten Ausführungsform wird ein Trennelement verwendet, das 1 bis 100 µg, besonders bevorzugt 5 bis 50 µg, ganz besonders bevorzugt 20 bis 25 µg ADP enthält.

Das verwendete Trennelement enthält weiterhin einen Aktivator von intrazellulären Adenylatzyklasen, bevorzugterweise aus der Gruppe Prostaglandin E1 (PGE 1), Forskolin und dessen wasserlösliche Derivate, Prostaglandin 12 und dessen stabile Derivate, Iloprost und Cicaprost. In einer bevorzugten Ausführungsform wird ein Trennelement verwendet, das 1 bis 1000 ng, besonders bevorzugt 3 bis 20 ng Prostaglandin E1 enthält. In einer anderen bevorzugten Ausführungsform wird ein Trennelement verwendet, das 0,1 bis 10 µg, besonders bevorzugt 0,5 bis 5 µg Forskolin enthält.

In einer besonders bevorzugten Ausführungsform des Verfahrens wird ein Trennelement verwendet, das ADP und Prostaglandin E1 enthält.

Außerdem enthält des Trennelement zusätzlich Calciumionen, bevorzugterweise in Form von Calciumchlorid Dihydrat. In einer bevorzugten Ausführungsform wird ein Trennelement verwendet, das 50 bis 200 µg, besonders bevorzugt 100 bis 150 µg, ganz besonders bevorzugt 125 µg Calciumionen in Form von Calciumchlorid Dihydrat enthält.

Es wurde gefunden, dass in Gegenwart von Calciumionen die thrombozytenaggregationshemmende Wirkung der Acetylsalicylsäure (ASS) so stark minimiert wird, dass eine präzise Bestimmung der thrombozytenaggregationshemmenden (antithrombotischen) Wirkung anderer Thrombozytenaggregationshemmer, wie z. B. von P2Y(12)-Antagonisten wie Clopidogrel, auch in solchen Proben möglich ist, die ASS enthalten. Ein Trennelement, das Calciumionen enthält, ist in dem erfindungsgemäßen Verfahren insbesondere dann zu verwenden, wenn die zu untersuchende Vollblutprobe mit einem Calcium-bindenden Antikoagulanz antikoaguliert ist. Ist die zu untersuchende Vollblutprobe mit einem Nicht-Calcium-bindenden Antikoagulanz antikoaguliert, wie z. B. mit einem direkten Thrombin- oder Faktor Xa-Inhibitor, so ist die endogen in der Probe enthaltene Calciumionenkonzentration ausreichend, um eine ASS-induzierte Thrombozytendysfunktion zu reduzieren. Nichtsdestotrotz kann auch in diesen Fällen ein Trennelement, das Calciumionen enthält, verwendet werden.

Das erfindungsgemäße Verfahren wird besonders bevorzugt zur Bestimmung der antithrombotischen (thrombozytenaggregationshemmenden) Wirkung eines P2Y(12)-Antagonisten verwendet, inbesondere zur Bestimmung eines P2Y(12)-Antagonisten aus der Gruppe: Clopidogrel, Ticlopidin, Prasugrel (synonym: CS-747) und andere Thienopyridine, AR-C67085MX (2-Propylthio-D-β,γ-dichlormethylen-Adenosin-5'-triphosphat), Cangrelor (synonym: AR-C69931 MX, N⁶-[2-(methylthio)-ethyl]-2-(3,3,3-trifluoropropyl)thio-5'-Adenylsäure), C1330-7 (N¹-(6-Ethoxy-1,3-benzothiazol-2-yl-2-(7-ethoxy-4-hydroxy-2,2-dioxo-2H-2-⁶benzo[4,5][1,3]thiazolo[2,3-c][1,2,4]thiadiazin-3-yl)-2-oxo-1-ethansulfonamid), AZD6140 (Nukleosidanalogon), MRS2395 (2,2-Dimethyl-propionsäure 3-(2-chloro-6-methylaminopurin-9-yl)-2-(2,2-dimethylpropionyloxymethyl)-propylester) und 2-MeSAMP (2-Methylthioadenosin-5'-monophospat).

Überraschenderweise wurde weiterhin gefunden, dass das erfindungsgemäße Verfahren ebenso zur Bestimmung der antithrombotischen (thrombozytenaggregationshemmenden) Wirkung eines P2Y(1)-Antagonisten verwendet werden kann. Insbesondere kann das Verfahren verwendet werden zur Bestimmung der antithrombotischen Wirkung von P2Y(1)-Antagonisten aus der Gruppe: MRS 2179 [2'-Deoxy-N⁶-methyladenosin 3',5'-diphosphat-Diammoniumsalz], MRS 2279 [(N)-methanocarba-N⁶-methyl-2-chloro-2'-deoxyadenosin-3',5'-bisphosphat], MRS 2500 [2-lodo-N⁶-methyl-(N)-methanocarba-2'-deoxyadenosin-3',5'-bisphosphat], A2P5P [Adenosin-2',5'-bisphosphat], A3P5P [Adenosin-3',5'-bisphosphat] und A3P5PS [Adenosin-3'-phosphat,5'-phosphosulfat].

Ein weiterer Gegenstand der vorliegenden Erfindung betrifft eine Vorrichtung, die sich zur Bestimmung der Thrombozytenfunktion in einer Vollblutprobe eignet, wobei die Vorrichtung verschiedene Elemente enthält: a) eine Haltekammer zum Halten der Probe; b) eine Kapillare, durch die das Blut von der Haltekammer in eine Messkammer geleitet wird; c) eine Messkammer, die durch ein Trennelement in zwei Kompartimente unterteilt ist, wobei das erste Kompartiment das Blut aus der Kapillare aufnimmt; d) ein Trennelement, das die Messkammer in zwei Kompartimente unterteilt und das eine Öffnung aufweist, durch welche das Blut aus dem ersten Kompartiment in das zweite Kompartiment fließen kann. Die Vorrichtung zeichnet sich dadurch aus, dass das Trennelement einen Aktivator von Adenosin-Rezeptoren, einen Aktivator von intrazellulären Adenylatzyklasen, sowie Calciumionen, bevorzugterweise in Form von Calciumchlorid Dihydrat.

Das Trennelement ist eine poröse oder nicht-poröse Stützmatrix für einen Aktivator von Adenosin-Rezeptoren und einen Aktivator von intrazellulären Adenylatzyklasen und gegebenenfalls für Calciumionen. Bevorzugterweise ist das Trennelement in Form einer Membran ausgestaltet. Das bevorzugte Material ist flüssigkeitsabsorbierend, so dass die vorgenannten Substanzen in gelöster Form aufgebracht werden können. Besonders bevorzugte Materialien sind Celluloseester, Keramik, Nylon, Polypropylen, Polyethersulfon und Polyvinylidenfluorid (PVDF). Bevorzugterweise wird das mit den gewünschten Substanzen benetzte oder durchtränkte Trennelement getrocknet. Durch den Kontakt des Blutes mit dem Trennelement werden die Substanzen aus dem Trennelement gelöst und vermischen sich mit der Blutprobe.

Das Trennelement weist eine bevorzugt kreisförmige Öffnung auf, die der Stützmatrix vorzugsweise durch Ausstanzen beigebracht wurde. Der Durchmesser der Öffnung im Trennelement ist derart bemessen, dass unter den Bedingungen des jeweiligen Verfahrens ein Thrombozytenpfropf gebildet werden kann, der die Öffnung verschließen und dadurch den Blutfluss stoppen kann. Vorzugsweise weist die Öffnung im Trennelement einen Durchmesser zwischen ungefähr 100 µm und ungefähr 200 µm auf. Besonders bevorzugt beträgt der Durchmesser der Öffnung im Trennelement etwa 100 µm.

Bevorzugterweise ist die erfindungsgemäße Vorrichtung so konstruiert, dass mit Hilfe eines Instruments, das mit Bauteilen der Vorrichtung interagiert, ein Unterdruck in der Vorrichtung erzeugt werden kann, der bewirkt, dass ein Blutfluss aus der Haltekammer durch die Kapillare in die Messzelle und durch die Öffnung des Trennelements erfolgt.

Die vorliegende Erfindung betrifft weiterhin die Verwendung einer erfindungsgemäßen Vorrichtung in einem Verfahren zur Bestimmung der Thrombozytenfunktion. Eine bevorzugte Verwendung einer erfindungsgemäßen Vorrichtung betrifft die Verwendung zur Bestimmung der antithrombotischen Wirkung eines P2Y(12)-Antagonisten. Eine andere bevorzugte Verwendung einer erfindungsgemäßen Vorrichtung betrifft die Verwendung zur Bestimmung der antithrombotischen Wirkung eines P2Y(1)-Antagonisten.

Die folgenden Ausführungsbeispiele dienen der Veranschaulichung des erfindungsgemäßen Verfahrens und sind nicht als Einschränkung zu verstehen.

### Figuren

### Figur 1

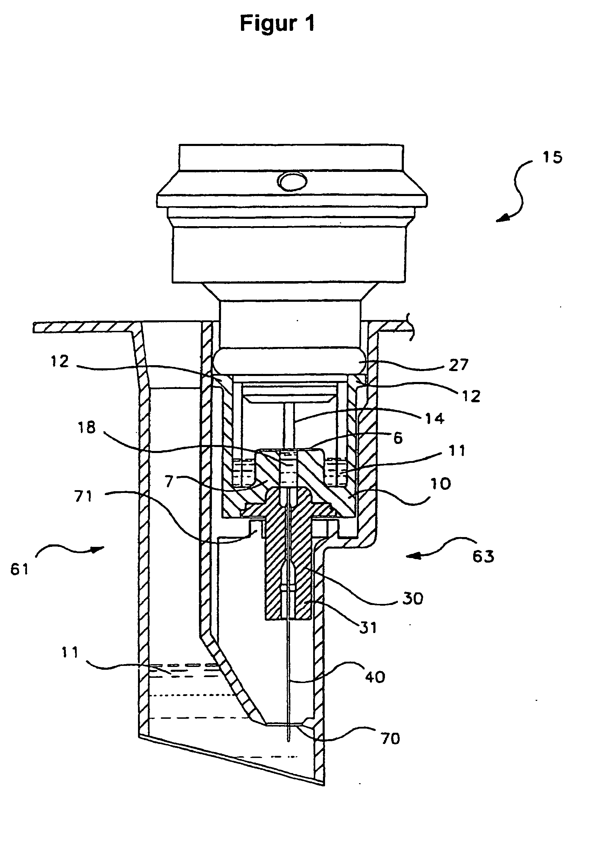

Figur 1 zeigt beispielhaft, wie eine erfindungsgemäße Vorrichtung zur Bestimmung der Thrombozytenfunktionen ausgestaltet sein kann. Gezeigt ist eine Testkartusche gemäß WO 97/34698 im Längsschnitt, die zur Durchführung des erfindungsgemäßen Verfahrens in einem passenden Instrument platziert ist und in welche ein Vakuumgerät (15) ragt, das für die Erzeugung des Unterdrucks verantwortlich ist. Das Vakuumgerät (15) weist dazu eine Ringdichtung (27) auf, die dichtend auf dem umlaufenden Rand (12) des Probenbechers (10) liegt. Die Testkartusche weist ein Gehäuse auf, das eine Haltekammer (61) und eine Testkammer (63) bildet. Die Testkammer (63) ist zur Aufnahme eines Probenbechers (10) ausgebildet, dessen Lumen auch als Messkammer bezeichnet werden kann. Der Probenbecher (10) stützt ein mit Reagenzien behandeltes Trennelement (6) mit einer zentralen Öffnung (Apertur) und einen Kapillaransatz (30, 31), der eine Verbindung der Kapillare (40) mit dem Probenbecher (10) herstellt. Haltekammer (61) und Testkammer (63) sind durch ein durchdringbares Element (70) getrennt. Die Figur zeigt eine Phase aus dem Testzyklus, nachdem das Vakuumgerät (15) den Probenbecher (10) berührt und nach unten bewegt hat, so dass der Boden des Probenbechers (10) das Stützelement (71) berührt hat und die Kapillare (40) das durchdringbare Element (70) durchdrungen hat und in die Probe (11) eingedrungen ist. Das Instrument erzeugt nun einen Unterdruck im Probenbecher (10), wodurch die Probe (11) durch die Kapillare (40) in das erste Kompartiment (18) der Messkammer und anschließend durch die Öffnung im Trennelement (6) nach oben gesogen wird.

### Figur 2

Diagramm zur Darstellung der Verschlusszeiten (in Sekunden [s]), die für normale, unbehandelte Vollblutproben (Kontrolle) und für Vollblutproben, die mit dem P2Y(12)-Antagonisten MRS 2395 und/oder dem COX-1-Inhibitor Acetylsalicylsäure (ASS) in vitro versetzt wurden (siehe Beispiel 2). Es wurden mit Natriumcitrat antikoagulierte Vollblutproben von 11 gesunden Spendern verwendet. Auf der linken Seite des Diagramms sind die Mittelwerte und die Standardabweichungen der Verschlusszeiten dargestellt, die mit erfindungsgemäßen ADP/PGE1/Calcium-Testkartuschen ermittelt wurden (Cut-off: 81 Sekunden). Auf der rechten Seite des Diagramms sind die Mittelwerte und die Standardabweichungen der Verschlusszeiten dargestellt, die zum Vergleich mit herkömmlichen Kol/Epi-Testkartuschen ermittelt wurden (Cut-off: 158 Sekunden). Der Leistungsvergleich der beiden Messzellentypen zeigt, dass bei Verwendung einer erfindungsgemäßen ADP/PGE1-Messzelle in Proben, die mit dem P2Y(12)-Antagonisten MRS 2395 versetzt wurden, Verschlusszeiten gemessen werden, die deutlich über dem oberen Referenzwert (Cut-off) liegen, während die gleichen Proben bei Verwendung einer Kol/Epi-Messzelle zu einem größeren Teil unterhalb des oberen Referenzwertes (Cut-off) liegen. Das bedeutet, dass das erfindungsgemäße Verfahren zur Bestimmung der Thrombozytenfunktion, eine sensitivere Bestimmung einer P2Y(12)-Antagonisten-induzierten Thrombozytendysfunktion ermöglicht als das Vergleichsverfahren aus dem Stand der Technik.

### Figur 3

Diagramm zur Darstellung der Verschlusszeiten (in Sekunden [s]), die für normale, unbehandelte Vollblutproben (Kontrolle) und für Vollblutproben, die mit dem P2Y(12)-Antagonisten MRS 2395, dem P2Y(1)-Antagonisten MRS 2179 oder dem COX-1-Inhibitor Acetylsalicylsäure (ASS) in vitro versetzt wurden (siehe Beispiel 4). Es wurden mit PPACK antikoagulierte Vollblutproben von 10 gesunden Spendern verwendet. Im Diagramm sind die Mittelwerte und die Standardabweichungen der Verschlusszeiten dargestellt, die mit erfindungsgemäßen ADP/PGE1-Testkartuschen ermittelt wurden (Cut-off: 90 Sekunden). Der Leistungsbewertung zeigt, dass bei Verwendung einer erfindungsgemäßen ADP/PGE1-Messzelle in Proben, die mit dem P2Y(12)-Antagonisten MRS 2395 oder dem P2Y(1)-Antagonisten MRS 2179 versetzt wurden, Verschlusszeiten gemessen werden, die deutlich über dem oberen Referenzwert (Cut-off) liegen, während die mit dem COX-1 Inhibitor Acetylsalicylsäure versetzten Proben keine Verlängerung der Verschlusszeiten zeigen und daher unter dem Cut-off liegen.

### Beispiele

### Beispiel 1: Herstellung einer erfindungsgemäßen ADP/PGE1/Calcium-Messzelle

Zur Herstellung eines Trennelements für eine erfindungsgemäße Testkartusche wurde eine Polyethersulfon-Filtermembran (Supor^{®}-Membran, Pall GmbH, Dreieich, Deutschland) in Streifen geschnitten. 1 µL einer Lösung, die 7 µg/µL ADP (Adenosin-5'-diphosphat Kaliumsalz - 2H₂O, Sigma-Aldrich Chemie GmbH, Steinheim, Deutschland) und 5 ng/pL PGE1 (Prostaglandin E1, Sigma-Aldrich Chemie GmbH, Steinheim, Deutschland) und 367,5 µg/µL CaCl₂ · 2H₂O (entspricht 100 µg/µL Ca²⁺-Ionen) enthielt, wurde punktförmig auf die Membran pipettiert, und die Membran wurde getrocknet. Anschließend wurde in die Mitte des mit den Reagenzien beschichteten Bereichs der Membran eine kreisförmige Öffnung (Apertur) mit einem Durchmesser von 100 µm gestanzt. Die so hergestellte Membran wurde als Trennelement in die Messkammer einer PFA-100^{®}-Testkartusche (Dade Behring Marburg GmbH, Marburg, Deutschland) eingesetzt.

### Beispiel 2: Verwendung einer erfindungsgemäßen ADP/PGE1/Calcium-Messzelle zur Bestimmung der antithrombotischen Wirkung eines P2Y(12)-Antagonisten in vitro

### 2a) Probenvorbereitung

11 gesunden Spendern wurde venöses Blut entnommen und mit Natriumcitrat antikoaguliert (3,2 % gepuffertes Na-Citrat).

Aliquots der Citratvollblutproben wurden in vitro mit dem P2Y(12)-Antagonisten MRS 2395 (Sigma-Aldrich Chemie GmbH, Steinheim, Deutschland) versetzt. Dazu wurde eine ethanolische MRS 2395-Stammlösung (15 mg/mL) mit den Vollblutproben vermischt, so dass eine Endkonzentration von 100 µmol/L erreicht wurde.

Weitere Aliquots der Citratvollblutproben wurden in vitro mit dem COX-1-Inhibitor Acetylsalicylsäure (kurz: ASS; Sigma-Aldrich Chemie GmbH, Steinheim, Deutschland) versetzt. Dazu wurde eine wässrige ASS-Stammlösung (1 mg/mL) mit den Vollblutproben vermischt, so dass eine Endkonzentration von 30 µmol/L erreicht wurde.

Weitere Aliquots der Citratvollblutproben wurden in vitro mit MRS 2395 und mit ASS versetzt, so dass die vorgenannten Endkonzentrationen erreicht wurden.

Nach Zugabe der Reagenzien wurden die Blutproben für 5 Minuten bei Raumtemperatur inkubiert.

### 2b) Bestimmung der antithrombotischen Wirkung von MRS 2395 mittels ADP induzierter Lichttransmissionaggregometrie (nach Born)

Um zu überprüfen, ob die MRS 2395-behandelten Proben tatsächlich eine verminderte Thrombozytenaggregation zeigen, wurde aus Aliquots der unter Beispiel 2a) beschriebenen unbehandelten und MRS 2395-behandelten Vollblutproben plättchenreiches (PRP) und plättchenarmes Plasma (PPP) hergestellt, und die Proben wurden anschließend mit 2 µM ADP versetzt. Die PPP-Proben wurden als Leerwertkontrollen verwendet. Die photometrische Messung der Aggregationsreaktion wurde in dem automatischen Gerinnungsgerät BCT^{®} (Dade Behring Marburg GmbH, Marburg, Deutschland) unter kontinuierlichem Rühren (600 U/min) vorgenommen. Die Thrombozytenaggregation der MRS 2395-behandelten Proben war gegenüber der Thrombozytenaggregation der unbehandelten Proben im Mittel um 27 % vermindert.

### 2c) Bestimmung der antithrombotischen Wirkung von MRS 2395 mit Hilfe des erfndungsgemäßen Verfahrens unter Flussbedingungen

Zur Bestimmung der Verschlusszeit als Maß für die Thrombozytenfunktion wurden die unter Beispiel 2a) beschriebenen Vollblutproben mit Hilfe der unter Beispiel 1 beschriebenen, erfindungsgemäßen ADP/PGE1/Calcium-Testkartuschen in einem PFA-100® Gerät (Platelet Function Analyzer-100, Dade Behring Marburg GmbH, Marburg, Deutschland) untersucht. Dazu wurden 700 µL einer Blutprobe in die Haltekammer der vortemperierten Testkartusche (+37 °C) gegeben und für 3 Minuten bei +37 °C im Gerät inkubiert. Anschließend wurde durch das Gerät ein Unterdruck von -40 mbar erzeugt, wodurch das Blut aus der Haltekammer durch eine Kapillare (Durchmesser 200 µm) und schließlich durch eine Öffnung (Apertur) des Trennelements in der Messkammer gesaugt wurde. Als Verschlusszeit wurde die Zeit bestimmt, die bis zum Verschluss der Apertur durch Bildung eines Blutgerinnsels benötigt wurde. Jede der untersuchten Proben wurde doppelt bestimmt, und als Messwert wurde der Mittelwert einer Doppelbestimmung verwendet.

Zu Vergleichszwecken wurden die unter Beispiel 2a) beschriebenen Vollblutproben parallel mit einer bekannten Kol/Epi PFA-100^{®}-Testkartusche (2 µg Kollagen und 10 µg Epinephrin auf der Membran; 150 µm Aperturdurchmesser; Dade Behring Marburg GmbH, Marburg, Deutschland) im PFA-100^{®}-Gerät untersucht.

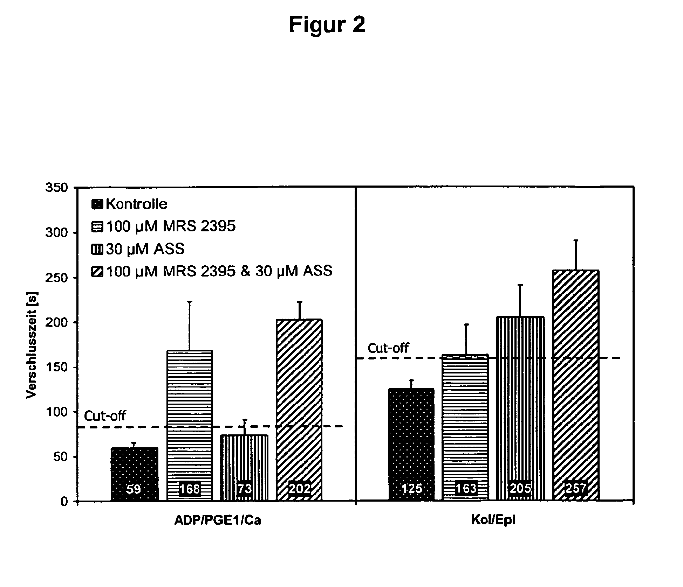

Die Ergebnisse der Untersuchungen sind in Figur 2 und der dazugehörigen Figurenbeschreibung zusammengefasst.

In Tabelle 1 ist im Einzelnen aufgeführt, für wieviele der jeweils 11 MRS 2395- und/oder Acetylsalicylsäure-behandelten Proben mit Hilfe der erfindungsgemäßen ADP/PGE1- bzw. mit Hilfe der konventionellen Kol/Epi-Messzelle eine Verschlusszeit oberhalb des Cut-Offs gemessen wurde. In 9 von 11 MRS 2395-behandelten Proben wird mit Hilfe des erfindungsgemäßen Verfahrens eine abnormal verminderte Thrombozytenaggregation gemessen, während mit Hilfe des konventionellen Verfahrens nur 4 von 11 Proben als abnormal eingestuft werden. Das bedeutet, dass das erfindungsgemäße Verfahren eine erhöhte Sensitivität für eine P2Y(12)-Antagonisten-induzierte Thrombozytendysfunktion aufweist. Weiterhin vorteilhaft ist, dass das erfindungsgemäße Verfahren in Gegenwart freier Calciumionen ein sehr geringe Sensitivität für Acetylsalicylsäure aufweist. Nur in einer von 11 Acetylsalicylsäure-behandelten Proben wird mit Hilfe des erfindungsgemäßen Verfahrens eine abnormal verminderte Thrombozytenaggregation gemessen, während hingegen mit der konventionellen Kol/Epi-Messzelle 8 der 11 Acetylsalicylsäure-behandelten Proben als abnormal bestimmt werden. Proben, die mit MRS 2395 und mit Acetylsalicylsäure behandelt sind, werden mit Hilfe des konventionellen Verfahrens zu 100 % als abnormal eingestuft, während das erfindungsgemäße Verfahren nur 9 von 11 Proben (wie auch bei alleiniger Zugabe von MRS 2395) als abnormal einstuft. Das erfindungsgemäße Verfahren eignet sich also aufgrund seiner hohen Sensitivität für P2Y(12)-Antagonisten-induzierte Thrombozytendysfunktionen und seiner geringen Sensitivität für Acetylsalicylsäureinduzierte Thrombozytendysfunktionen, zur Differenzierung der beiden Antithrombotika-Klassen.

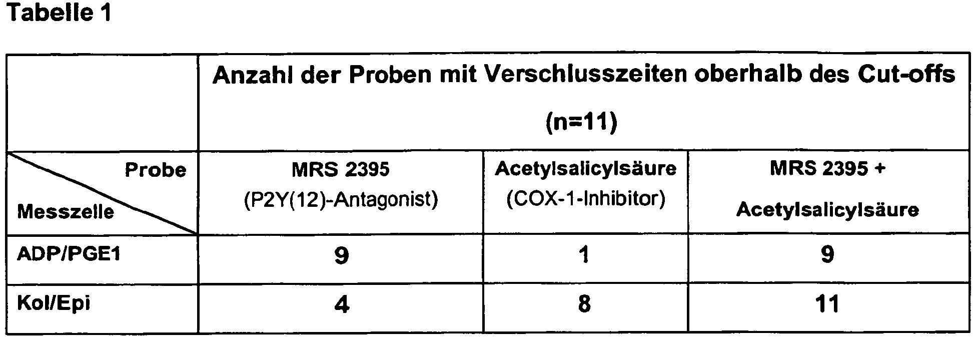

### 2d) Ermittlung der Referenzbereiche für Kol/Epi- und ADP/PGE-Testkartuschen

Gesunden Spendern wurde venöses Blut entnommen und mit Natriumcitrat antikoaguliert (3,2 % gepuffertes Na-Citrat). Für jede Vollblutprobe wurde die Verschlusszeitbestimmung im PFA-100^{®}-Gerät durchgeführt. Mit einer Kol/Epi PFA-100^{®}-Testkartusche [siehe Beispiel 2c)] wurden Proben von 186 Spendern in Doppelbestimmung untersucht. Mit einer erfindungsgemäßen ADP/PGE1/Calcium-Testkartusche [siehe Beispiele 1 und 2c)] wurden Proben von 159 Spendern in Doppelbestimmung untersucht.

Der Referenzbereich (Normalbereich) für die Kol/Epi-Verschlusszeit bzw. für die ADP/PGE1-Verschlusszeit wurde festgelegt, indem der Messwertebereich bestimmt wurde, in dem 90 % der Messwerte lagen, die für die gesunden Probanden bestimmt worden waren (90 % Zentralinterval der Normalverteilung aller Messungen). Damit ergaben sich folgende Referenzbereiche für die Verschlusszeiten:

| | | |
|---|---|---|
| Kol/Epi | 70 - 158 | Sekunden |
| ADP/PGE1 | 46 - 81 | Sekunden. |

Als Cut-Off, d. h. als Grenzwert, für eine Thrombozytendysfunktion wurde die obere Referenzgrenze des Referenzbereichs festgelegt. Weicht die Verschlusszeit einer Patientenprobe vom Referenzbereich ab, kann dies für eine Störung der Thrombozytenfunktion sprechen. Das heisst, Kol/Epi-Verschlusszeiten, die größer als 158 Sekunden sind bzw. ADP/PGE1- Verschlusszeiten, die größer als 81 Sekunden sind, weisen auf das Vorliegen einer Thrombozytendysfunktion im Sinne einer verminderten Aggregationsfähigkeit hin.

### Beispiel 3: Verwendung einer erfindungsgemäßen ADP/PGE1-Messzelle zur Bestimmung der antithrombotischen Wirkung des P2Y(12)-Antagonisten Clopidogrel ex vivo

13 Patienten, die an einer peripheren arteriellen Verschlusskrankheit litten und über einen Zeitraum von mindestens 4 Wochen mit einer täglichen Dosis von 75 mg Clopidogrel (Plavix^{®}, Sanofi-Aventis) als einzigem Antithrombotikum behandelt worden waren, wurde venöses Blut entnommen und mit Natriumcitrat antikoaguliert (3,8 % gepuffertes Na-Citrat). Die Proben wurden mit Hilfe verschiedener Verfahren zur Bestimmung der Thrombozytenfunktion untersucht:
1. erfindungsgemäß unter Flussbedingungen und Verwendung einer ADP/PGE1/Calcium PFA-100^{®}-Testkartusche (siehe Beispiele 1 und 2c), Cut-off: > 81 Sekunden;
2. unter Flussbedingungen und Verwendung einer PFA-100®-Testkartusche, die sich von der erfindungsgemäßen Testkartusche gemäß Beispiel 1 dadurch unterschied, dass sie kein PGE1 enthielt, Cut-off: > 78 Sekunden;
3. mittels ADP induzierter Lichttransmissionaggregometrie (nach Born) bei Zugabe von 2 µM ADP (siehe Beispiel 2b), Cut-off: < 40 % Lichttransmission bei Testende;
4. mittels ADP induzierter Lichttransmissionaggregometrie (nach Born) bei Zugabe von 5 µM ADP (siehe Beispiel 2b), Cut-off: < 40 % Lichttransmission bei Testende. Dieses Verfahren wird von Sanofi-Aventis, dem Hersteller des Clopidogrel-Präparats Plavix^{®} zur Bestimmung der antithrombotischen Wirkung des Medikaments empfohlen.

Die Cut-offs für die einzelnen Testverfahren waren durch Vorversuche mit Vollblut- bzw. Plasmaproben normaler Spender bestimmt worden.

Tabelle 2 führt im Einzelnen auf, mit welchem der vier Verfahren in welcher der 13 Patientenproben eine antithrombotische Wirkung von Clopidogrel nachgewiesen werden konnte. "+" bedeutet, es konnte ein antithrombotischer Effekt nachgewiesen werden. "-" bedeutet, es konnte kein antithrombotischer Effekt nachgewiesen werden. "0" bedeutet, dass die Doppelbestimmung widersprüchliche Ergebnisse lieferte, d. h. einen Wert oberhalb und einen Wert unterhalb des Cut-offs.

**Tabelle 2**

| | **Verfahren** | **1** | **2** | **3** | **4** | **5** | **6** | **7** | **8** | **9** | **10** | **11** | **12** | **13** | **Sensitivität** |
|---|---|---|---|---|---|---|---|---|---|---|---|---|---|---|---|
| **1.)** | **ADP/PGE1** | **-** | **0** | **+** | **+** | **+** | **-** | **+** | **+** | **+** | **+** | **+** | **+** | **+** | **77%** |
| **2.)** | **ADP** | **-** | **-** | **+** | **+** | **+** | **-** | **-** | **-** | **+** | **-** | **+** | **-** | **+** | **46%** |
| **3.)** | **Aggregometrie 2 µM ADP** | **-** | **+** | **-** | **+** | **+** | **-** | **-** | **+** | **+** | **+** | **+** | **+** | **+** | **69%** |
| **4.)** | **Aggregometrie 5 µM ADP** | **-** | **-** | **-** | **+** | **+** | **-** | **-** | **+** | **+** | **+** | **+** | **+** | **+** | **62%** |

Mit Hilfe des erfindungsgemäßen Verfahrens konnte bei 10 von 13 Patienten (77 %) der antithrombotische Effekt der Einnahme von Clopidogrel nachgewiesen werden. Bei einem Patienten (Patient Nr. 2) lieferte die Doppelbestimmung widersprüchliche Ergebnisse, während bei 2 Patienten (Patienten Nr. 1 und 6) keine verminderte Thrombozytenaggregation nachgewiesen werden konnte. Allerdings konnte bei diesen beiden Patienten mit keiner der angewandten Methoden Effekt der Einnahme von Clopidogrel nachgewiesen werden.

Das erfindungsgemäße Verfahren ist sensitiver gegenüber der durch Clopidogrel induzierten Thrombozytendysfunktion als die Standardmethode der ADP induzierten Lichttransmissionsaggregometrie nach Born und sensitiver als das Verfahren, bei dem eine Testkartusche verwendet wird, die ADP, aber kein PGE1 enthält.

### Beispiel 4: Verwendung einer erfindungsgemäßen ADP/PGE1-Messzelle zur Bestimmung der antithrombotischen Wirkung eines P2Y(12)- und eines P2Y(1)-Antagonisten in vitro

### 4a) Probenvorbereitung

10 gesunden Spendern wurde venöses Blut entnommen und mit 75 µM PPACK antikoaguliert.

Aliquots der Vollblutproben wurden in vitro mit dem P2Y(12)-Antagonisten MRS 2395 (Sigma-Aldrich Chemie GmbH, Steinheim, Deutschland) versetzt. Dazu wurde eine ethanolische MRS 2395-Stammlösung (15 mg/mL) mit den Vollblutproben vermischt, so dass eine Endkonzentration von 150 µmol/L erreicht wurde.

Weitere Aliquots der Vollblutproben wurden in vitro mit dem COX-1-Inhibitor Acetylsalicylsäure (kurz: ASS; Sigma-Aldrich Chemie GmbH, Steinheim, Deutschland) versetzt. Dazu wurde eine wässrige ASS-Stammlösung (1 mg/mL) mit den Vollblutproben vermischt, so dass eine Endkonzentration von 30 µmol/L erreicht wurde.

Weitere Aliquots der Vollblutproben wurden in vitro mit dem P2Y(1)-Antagonisten MRS 2179 (Sigma-Aldrich Chemie GmbH, Steinheim, Deutschland) versetzt. Dazu wurde eine wässrige MRS 2179-Stammlösung (1 mg/mL) mit den Vollblutproben vermischt, so dass eine Endkonzentration von 75 µmol/L erreicht wurde.

Nach Zugabe der Reagenzien wurden die Blutproben für 5 Minuten bei Raumtemperatur inkubiert.

### 4b) Bestimmung der antithrombotischen Wirkung von MRS 2395 und MRS 2179 mit Hilfe des erfindungsgemäßen Verfahrens unter Flussbedingungen

Zur Bestimmung der Verschlusszeit als Maß für die Thrombozytenfunktion wurden die unter Beispiel 4a) beschriebenen Vollblutproben mit Hilfe einer erfindungsgemäßen ADP/PGE1-Testkartuschen in einem PFA-100® Gerät (Platelet Function Analyzer-100, Dade Behring Marburg GmbH, Marburg, Deutschland) untersucht. Die verwendete erfindungsgemäße ADP/PGE1-Testkartusche wurde im wesentlichen, wie in Beispiel 1 beschrieben, hergestellt, allerdings ohne dass das Trennelement mit CaCl₂ · 2H₂O versetzt wurde. Die Testkartusche enthielt also 7 µg ADP und 5 ng PGE1, aber keine Calciumionen.

Es wurden 700 µL einer Blutprobe in die Haltekammer der vortemperierten Testkartusche (+37 °C) gegeben und für 3 Minuten bei +37 °C im Gerät inkubiert. Anschließend wurde durch das Gerät ein Unterdruck von -40 mbar erzeugt, wodurch das Blut aus der Haltekammer durch eine Kapillare (Durchmesser 200 µm) und schließlich durch eine Öffnung (Apertur) des Trennelements in der Messkammer gesaugt wurde. Als Verschlusszeit wurde die Zeit bestimmt, die bis zum Verschluss der Apertur durch Bildung eines Blutgerinnsels benötigt wurde. Jede der untersuchten Proben wurde doppelt bestimmt, und als Messwert wurde der Mittelwert einer Doppelbestimmung verwendet.

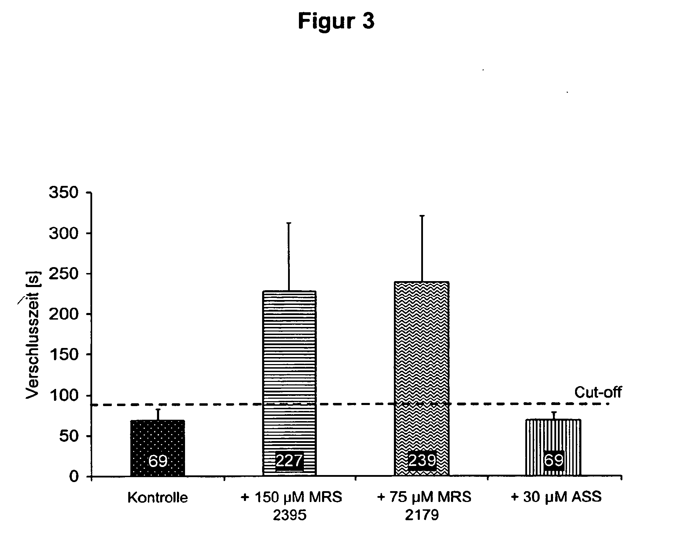

Die Ergebnisse der Untersuchungen sind in Figur 3 und der dazugehörigen Figurenbeschreibung zusammengefasst.

In Tabelle 3 ist im Einzelnen aufgeführt, für wieviele der jeweils 10 MRS 2395-, MRS 2179- oder Acetylsalicylsäure-behandelten Proben mit Hilfe der erfindungsgemäßen ADP/PGE1-Messzelle eine Verschlusszeit oberhalb des Cut-Offs gemessen wurde. In 9 von 10 MRS 2395-behandelten Proben und in 10 von 10 MRS 2179-behandelten Proben wurde mit Hilfe des erfindungsgemäßen Verfahrens eine abnormal verminderte Thrombozytenaggregation gemessen, während keine der mit Acetylsalicylsäure-behandelten Proben als abnormal eingestuft wurde. Das bedeutet, dass das erfindungsgemäße Verfahren eine hohe Sensitivität sowohl für eine durch einen P2Y(12)-Antagonisten induzierte als auch für eine durch einen P2Y(1)-Antagonisten induzierte Thrombozytendysfunktion aufweist. Weiterhin vorteilhaft ist, dass das erfindungsgemäße Verfahren in Gegenwart freier Calciumionen ein sehr geringe Sensitivität für Acetylsalicylsäure aufweist.

Das erfindungsgemäße Verfahren eignet sich also aufgrund seiner hohen Sensitivität für eine durch Antagonisten der ADP-Rezeptoren induzierte Thrombozytendysfunktionen und seiner geringen Sensitivität für Acetylsalicylsäureinduzierte Thrombozytendysfunktionen zur Differenzierung der beiden Antithrombotika-Klassen.

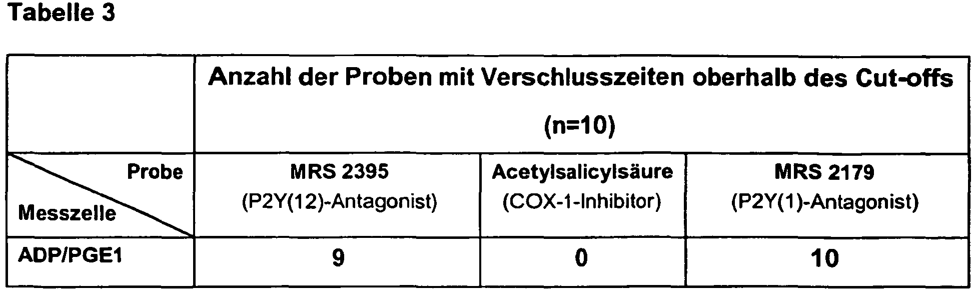

### 4c) Ermittlung des Cut-off für ADP/PGE-Testkartuschen

Wegen der Verwendung von PPACK als Antikoagulanz kann der mit Zitratvollblut ermittelte Cut-off der oben aufgeführten Beispiele nicht verwendet werden. Daher wurde der Referenzbereich für die ADP/PGE1-Verschlusszeit aus den 10 PPACKbehandelten Proben der gesunden Spender durch Bestimmung des 90 % Zentralintervals der Normalverteilung der Mittelwerte der Doppelbestimmungen ermittelt. Damit ergaben sich folgende Referenzbereiche für die Verschlusszeit:

| | | |
|---|---|---|
| ADP/PGE1 | 51-90 | Sekunden. |

Die obere Grenze des 90 % Zentralintervals wurde als Cut-off, d. h. als Grenzwert, für eine Thrombozytendysfunktion festgelegt.

## Patentansprüche

1. Verfahren zur Bestimmung der Thrombozytenfunktion in einer Vollblutprobe, wobei das Verfahren folgende Schritte umfasst:
a) Durchleiten des Blutes durch eine Kapillare und anschließend durch eine Öffnung eines Trennelements; und
b) Messen der Zeit, die für die Bildung eines Thrombozytenpfropfs an der Öffnung des Trennelements bis zum Verschluss der Öffnung benötigt wird;
**dadurch gekennzeichnet, dass**
das verwendete Trennelement
i) einen Aktivator von Adenosin-Rezeptoren,
ii) einen Aktivator von intrazellulären Adenylatzyklasen aus der Gruppe Prostaglandin E1, Forskolin, Prostaglandin 12 und dessen stabile Derivate Iloprost und Cicaprost, und
iii) Calciumionen
enthält.

2. Verfahren gemäß Anspruch 1 **dadurch gekennzeichnet, dass** das verwendete Trennelement einen Aktivator von Adenosin-Rezeptoren aus der Gruppe Adenosin-5'-diphosphat und 2-Methylthioadenosin-5'-diphosphat enthält.

3. Verfahren gemäß einem der Ansprüche 1 bis 2 **dadurch gekennzeichnet, dass** die Vollblutprobe mit einem direkten Thrombininhibitor antikoaguliert ist.

4. Verfahren gemäß einem der Ansprüche 1 bis 2 **dadurch gekennzeichnet, dass** die Vollblutprobe mit einem direkten Faktor Xa-Inhibitor antikoaguliert ist.

5. Verfahren gemäß einem der Ansprüche 1 bis 2 **dadurch gekennzeichnet, dass** die Vollblutprobe mit Citrat antikoaguliert ist.

6. Verwendung eines Verfahrens gemäß einem der Ansprüche 1 bis 5 zur Bestimmung der antithrombotischen Wirkung eines P2Y(12)-Antagonisten.

7. Verwendung gemäß Anspruch 6 zur Bestimmung der antithrombotischen Wirkung eines P2Y(12)-Antagonisten aus der Gruppe Clopidogrel, Ticlopidin, Prasugrel, AR-C67085MX, Cangrelor, C1330-7, MRS 2395 und 2-Methylthioadenosin-5'-monophosphat.

8. Verwendung eines Verfahrens gemäß einem der Ansprüche 1 bis 5 zur Bestimmung der antithrombotischen Wirkung eines P2Y(1)-Antagonisten.

9. Verwendung gemäß Anspruch 8 zur Bestimmung der antithrombotischen Wirkung eines P2Y(1)-Antagonisten aus der Gruppe MRS 2179, MRS 2279, MRS 2500, A2P5P, A3P5P und A3P5PS.

10. Vorrichtung zur Bestimmung der Thrombozytenfunktion in einer Vollblutprobe, wobei die Vorrichtung folgende Elemente enthält:
a) eine Haltekammer zum Halten der Probe;
b) eine Kapillare, durch die das Blut von der Haltekammer in eine Messkammer geleitet wird;
c) eine Messkammer, die durch ein Trennelement in zwei Kompartimente unterteilt ist, wobei das erste Kompartiment das Blut aus der Kapillare aufnimmt;
d) ein Trennelement, das die Messkammer in zwei Kompartimente unterteilt und das eine Öffnung aufweist, durch welche das Blut aus dem ersten Kompartiment in das zweite Kompartiment fließen kann;
**dadurch gekennzeichnet, dass**
das Trennelement
i) einen Aktivator von Adenosin-Rezeptoren,
ii) einen Aktivator von intrazellulären Adenylatzyklasen aus der Gruppe Prostaglandin E1, Forskolin, Prostaglandin I2 und dessen stabile Derivate Iloprost und Cicaprost, und
iii) Calciumionen
enthält.

11. Verwendung einer Vorrichtung gemäß Anspruch 10 in einem Verfahren zur Bestimmung der Thrombozytenfunktion.

12. Verwendung einer Vorrichtung gemäß einem der Ansprüche 10 und 11 in einem Verfahren zur Bestimmung der antithrombotischen Wirkung eines P2Y(12)-Antagonisten.

13. Verwendung einer Vorrichtung gemäß einem der Ansprüche 10 und 11 in einem Verfahren zur Bestimmung der antithrombotischen Wirkung eines P2Y(1)-Antagonisten.

## Claims

1. Method for the determination of platelet function in a whole blood sample wherein the method has the following steps:
a) passing the blood though a capillary and then through an opening of a divider; and
b) measurement of the time that is required for the formation of a thrombus at the opening of the divider up to closure of the opening;
**characterized in that**
the divider used comprises
i) an activator of adenosine receptors,
ii) an activator of intracellular adenylate cyclases from the group prostaglandin E1, forskolin, prostaglandin 12 and its stable derivatives, illoprost and cicaprost, and
iii) calcium ions.

2. Method according to Claim 1, **characterized in that** the divider used comprises an activator of adenosine receptors from the group adenosine-5'-diphosphate, 2-methylthioadenosine-5'-diphosphate.

3. Method according to one of the Claims 1 to 2, **characterized in that** the whole blood sample is anticoagulated with a direct thrombin inhibitor.

4. Method according to one of the Claims 1 to 2, **characterized in that** the whole blood sample is anticoagulated with a direct Factor Xa inhibitor.

5. Method according to one of the Claims 1 to 2, **characterized in that** the whole blood sample is anticoagulated with citrate.

6. Use of a method according to one of the Claims 1 to 5 for the determination of the antithrombotic action of a P2Y(12) antagonist.

7. Use according to Claim 6 for the determination of the antithrombotic action of a P2Y(12) antagonist from the group clopidogrel, ticlopidine, prasugrel, AR-C67085MX, cangrelor, C1330-7, MRS 2395, and 2-methylthioadenosine-5'-monophosphate.

8. Use of a method according to one of the Claims 1 to 5 for the determination of the antithrombotic action of a P2Y(1) antagonist.

9. Use according to Claim 8 for the determination of the antithrombotic action of a P2Y(1) antagonist from the group MRS 2179, MRS 2279, MRS 2500, A2P5P, A3P5P, and A3P5PS.

10. Device for the determination of platelet function in a whole blood sample wherein the device comprises the following elements:
a) a reservoir for holding the sample;
b) a capillary through which the blood is passed from the reservoir into a measurement chamber;
c) a measurement chamber that is divided into two compartments by a divider, wherein the first compartment receives the blood from the capillary;
d) a divider that divides the measurement chamber into two compartments and has an opening through which the blood can flow from the first compartment into the second compartment;
**characterized in that**
the divider comprises
i) an activator of adenosine receptors,
ii) an activator of intracellular adenylate cyclases, from the group prostaglandin El, forskolin, prostaglandin 12 and its stable derivatives, illoprost and cicaprost, and
iii) calcium ions

11. Use of a device according to Claim 10 in a method for the determination of platelet function.

12. Use of a device according to one of the Claims 10 and 11 in a method for the determination of the antithrombotic action of a P2Y(12) antagonist.

13. Use of a device according to one of the Claims 10 and 11 in a method for the determination of the antithrombotic action of a P2Y(1) antagonist.

## Revendications

1. Procédé de détermination de la fonction des thrombocytes dans un échantillon de sang total, le procédé comprenant les stades suivants :
a) on fait passer le sang dans un capillaire et ensuite dans une ouverture d'un élément de séparation ; et
b) on mesure le temps qui est nécessaire jusqu'à la fermeture de l'ouverture pour la formation d'un bouchon de thrombocytes à l'ouverture de l'élément de séparation,
**caractérisé en ce que**
l'élément de séparation utilisé contient
i) un activateur de récepteurs d'adénosine,
ii) un activateur d'adénylatecyclases intracellulaires choisi dans le groupe de la prostaglandine E1, de la forscholine, de la prostaglandine 12 et de leurs dérivés stables iloprost et cicaprost, et
iii) des ions calcium.

2. Procédé suivant la revendication 1, **caractérisé en ce que** l'élément de séparation utilisé contient un activateur de récepteurs d'adénosine choisi dans le groupe du 5'-diphosphate d'adénosine et du 5'-diphosphate de 2-méthylthioadénosine.

3. Procédé suivant l'une des revendications 1 à 2, **caractérisé en ce que** l'échantillon de sang total est anti-coagulé par un inhibiteur direct de thrombine.

4. Procédé suivant l'une des revendications 1 à 2, **caractérisé en ce que** l'échantillon de sang total est anti-coagulé par un inhibiteur direct du facteur Xa.

5. Procédé suivant l'une des revendications 1 à 2, **caractérisé en ce que** l'échantillon de sang total est anti-coagulé par un citrate.

6. Utilisation d'un procédé suivant l'une des revendications 1 à 5, pour la détermination de l'effet anti-thrombotique d'un antagoniste de P2Y(12).

7. Utilisation suivant la revendication 6, pour la détermination de l'effet anti-thrombotique d'un antagoniste de P2Y(12) choisi dans le groupe du clopidogrel, de la ticlopidine, du prasugrel, de l'AR-C67085MX, du cangrélor, du C1330-7, du MRS 2395 et du 5'-monophosphate de 2-méthylthioadénosine.

8. Utilisation d'un procédé suivant l'une des revendications 1 à 5, pour la détermination de l'effet anti-thrombotique d'un antagoniste de P2Y(1).

9. Utilisation d'un procédé suivant la revendication 8, pour la détermination de l'effet anti-thrombotique d'un antagoniste de P2Y(1) choisi dans le groupe du MRS 2179, du MRS 2279, du MRS 2500, de l'A2P5P, de l'A3P5P et de l'A3P5PS.

10. Dispositif de détermination de la fonction des thrombocytes dans un échantillon de sang total, le dispositif comprenant les éléments suivants :
a) une chambre de retenue pour retenir l'échantillon ;
b) un capillaire par lequel le sang passe de la chambre de retenue à une chambre de mesure,
c) une chambre de mesure, qui est subdivisée par un élément de séparation en deux compartiments, le premier compartiment recevant le sang venant du capillaire,
d) un élément de séparation, qui subdivise la chambre de mesure en deux compartiments et qui a une ouverture par laquelle le sang peut s'écouler du premier compartiment dans le deuxième compartiment ;
**caractérisé en ce que**
l'élément de séparation contient
i) un activateur de récepteurs d'adénosine,
ii) un activateur d'adénylatecyclase intracellulaires choisi dans le groupe de la prostaglandine E1, de la forscholine, de la prostaglandine 12 et de leurs dérivés stables iloprost et cicaprost, et
iii) des ions calcium.

11. Utilisation d'un dispositif suivant la revendication 10, dans un procédé de détermination de la fonction des thrombocyte.

12. Utilisation d'un dispositif suivant l'une des revendications 10 et 11, dans un procédé de détermination de l'effet anti-thrombotique d'un antagoniste de P2Y(12).

13. Utilisation d'un dispositif suivant l'une des revendications 10 et 11, dans un procédé de détermination de l'effet anti-thrombotique d'un antagoniste de P2Y(1).
